# EUROPÄISCHE PATENTANMELDUNG

(11) **EP 3 939 556 A1**
(43) Veröffentlichungstag der Anmeldung: **19.01.2022**
(21) Anmeldenummer: 21184182.0
(22) Anmeldetag: 07.07.2021
(51) Int. Cl.: A61G 13/10, A61G 10/00

(54) **TRANSPORTSYSTEM FÜR EINEN OPERATIONSSAAL UND TRANSPORTVERFAHREN**

(30) Priorität: 13.07.2020 DE 102020118375
(71) Anmelder: Mathys AG Bettlach, 2544 Bettlach (CH)
(72) Erfinder: Reinmann, Benjamin, 2544 Bettlach (CH); Mattmann.Oliver, 6300 Zug (CH)
(74) Vertreter: Körfer, Thomas

(57) **Zusammenfassung**

Es ist ein Transportsystem (10) für einen Operationssaal (15) gegeben. Dabei ist das Transportsystem (10) dazu eingerichtet, mindestens zwei zu behandelnde Personen jeweils zumindest teilweise durch das Innere des Operationssaals (15) gemäß einem Takt zu bewegen.

## Beschreibung

### GEBIET DER ERFINDUNG

Die Erfindung betrifft ein Transportsystem für einen Operationssaal sowie ein Transportverfahren.

### HINTERGRUND

Aufgrund steigender Anforderungen an die Kosteneffizienz bezüglich der Durchführung von chirurgischen Eingriffen wächst der Bedarf an entsprechenden Transportsystemen für Operationssäle sowie entsprechenden Transportverfahren, mithilfe derer eine besonders hohe Kosten- und Zeitersparnis realisiert werden kann.

Das Dokument US 10,363,102 B2 zeigt ein Verfahren zur Durchführung eines medizinischen Eingriffs in einem Operationssaal. Dieses Verfahren umfasst unter anderem den Schritt des Definierens eines vorbestimmten Betriebsplans, der mehrere Positionen eines Prozedurobjekts über die Zeit bedient, sowie den Schritt des Zuweisens eines gewünschten Zeitmeilensteins innerhalb des Verfahrens für jede Position des Objekts in dem vorgegebenen Betriebsplan. Nachteilhafterweise befindet sich gemäß dem vorgenannten Dokument ausschließlich ein Patient zur Behandlung in dem Operationssaal, wodurch eine Optimierung von Zeit- und Kostenersparnis schnell an ihre Grenzen stößt.

### AUFGABE UND LÖSUNG

Demnach ist es die Aufgabe der Erfindung, ein Transportsystem für einen Operationssaal sowie ein Transportverfahren bereitzustellen, mit deren Hilfe Operationen bzw. chirurgisch Eingriffe auf besonders zeit- und kosteneffiziente Weise durchgeführt werden können.

Die Aufgabe wird bezüglich des Transportsystem für einen Operationssaal durch die Merkmale des Patentanspruchs 1 gelöst. Die Aufgabe wird bezüglich des Transportverfahrens durch die Merkmale des Patentanspruchs 10 gelöst. Die Unteransprüche enthalten vorteilhafte Weiterbildungen.

### BESCHREIBUNG DER ERFINDUNG

Gemäß einem ersten Aspekt der Erfindung wird ein Transportsystem für einen Operationssaal geschaffen. Dabei ist das Transportsystem dazu eingerichtet, mindestens zwei zu behandelnde Personen jeweils zumindest teilweise durch das Innere des Operationssaals gemäß einem Takt zu bewegen.

Vorteilhafterweise kann so die Auslastung im Operationssaal sehr stark erhöht werden, wodurch Operationen bzw. chirurgisch Eingriffe auf besonders zeit- und kosteneffiziente Weise durchgeführt werden können.

Gemäß einer ersten bevorzugten Ausführungsform des ersten Aspekts der Erfindung ist das Transportsystem dazu eingerichtet, die mindestens zwei zu behandelnden Personen derart zu bewegen, dass jeweils zumindest temporär eine Relativbewegung zwischen zumindest einem Teil der mindestens zwei zu behandelnden Personen und zumindest einem Teil zur entsprechenden Behandlung erforderlicher Vorrichtungen und/oder Materialien entsteht.

Zusätzlich oder alternativ ist zumindest ein Teil der zur entsprechenden Behandlung erforderlichen Vorrichtungen und/oder Materialien zumindest teilweise im Inneren des Operationssaals statisch oder nahezu statisch lokalisiert.

Vorteilhafterweise kann aufgrund einer vordefinierten Lage der Vorrichtungen und/oder Materialien bzw. zumindest eines Teils davon ein Austausch derselben auf besonders einfache und effiziente Weise vorgenommen werden.

Gemäß einer zweiten bevorzugten Ausführungsform des ersten Aspekts der Erfindung ist das Transportsystem dazu eingerichtet, die mindestens zwei zu behandelnden Personen derart zu bewegen, dass zwischen zumindest einem Teil der mindestens zwei zu behandelnden Personen und mindestens einer behandelnden Person keine oder eine vernachlässigbare Relativbewegung entsteht.

Vorteilhafterweise kann eine behandelnde Person die entsprechende zu behandelnde Person zumindest teilweise begleiten. So kann beispielsweise der Personalbedarf reduziert werden, was zu einer höheren Kosteneffizienz führt.

Zusätzlich oder alternativ ist das Transportsystem dazu eingerichtet, die mindestens zwei zu behandelnden Personen derart zu bewegen, dass jeweils zumindest temporär eine Relativbewegung zwischen mindestens einer behandelnden Person und zumindest einem Teil der zur entsprechenden Behandlung erforderlichen Vorrichtungen und/oder Materialien entsteht, wobei im Falle der ersten bevorzugten Ausführungsform des ersten Aspekts der Erfindung, die vorgenannte Relativbewegung insbesondere der Relativbewegung gemäß dieser ersten bevorzugten Ausführungsform zumindest annähernd entspricht.

Weiter zusätzlich oder alternativ ist mindestens eine behandelnde Person im Inneren des Operationssaals statisch oder nahezu statisch lokalisiert. Vorteilhafterweise kann sich zumindest ein Teil des Personals auf wenige Arbeitsschritte im Rahmen der Operation fokussieren bzw. spezialisieren, wodurch diese besonders effizient ausgeführt werden können.

Gemäß einer weiteren bevorzugten Ausführungsform des ersten Aspekts der Erfindung ist das Transportsystem dazu eingerichtet, zumindest einen Teil zur entsprechenden Behandlung erforderlicher Vorrichtungen und/oder Materialien derart zu bewegen, dass jeweils zumindest temporär eine Relativbewegung zwischen zumindest einem Teil der mindestens zwei zu behandelnden Personen und zumindest einem Teil zur entsprechenden Behandlung erforderlicher Vorrichtungen und/oder Materialien entsteht. Vorteilhafterweise kann das erforderliche Equipment direkt zum Patienten transportiert werden, was entsprechende Beschaffungszeiten stark reduziert.

Zusätzlich oder alterativ ist das Transportsystem dazu eingerichtet, zumindest einen Teil zur entsprechenden Behandlung erforderlicher Vorrichtungen und/oder Materialien derart zu bewegen, dass jeweils zumindest temporär eine Relativbewegung zwischen mindestens einer behandelnden Person und zumindest einem Teil der zur entsprechenden Behandlung erforderlichen Vorrichtungen und/oder Materialien entsteht. Vorteilhafterweise kann das erforderliche Equipment direkt zum medizinischen Personal transportiert werden, was entsprechende Beschaffungszeiten stark reduziert und so die Kosteneffizienz erhöht.

Gemäß einer weiteren bevorzugten Ausführungsform des ersten Aspekts der Erfindung ist die entsprechende Bewegungsrichtung des zumindest einen Teils der zur entsprechenden Behandlung erforderlichen Vorrichtungen und/oder Materialien senkrecht oder nahezu senkrecht zu der entsprechenden Bewegungsrichtung mindestens eines Teils der mindestens zwei zu behandelnden Personen. Vorteilhafterweise wird so die Bewegungsfreiheit des medizinischen Personals nicht eingeschränkt, wodurch ein effizientes Arbeiten gewährleistet wird.

Gemäß einer weiteren bevorzugten Ausführungsform des ersten Aspekts der Erfindung weist das Transportsystem mindestens einen sterilen Bereich, vorzugsweise mindestens einen sterilen Bereich im Inneren des Operationssaals, auf. Zusätzlich oder alternativ wiest das Transportsystem mindestens einen unsterilen Bereich, vorzugsweise mindestens einen unsterilen Bereich außerhalb des Operationssaals, auf. Vorteilhafterweise kann sich das Transportsystem über den Operationssaal hinaus erstrecken, wodurch eine entsprechende Zeitersparnis insgesamt erhöht werden kann.

Gemäß einer weiteren bevorzugten Ausführungsform des ersten Aspekts der Erfindung weist das Transportsystem mindestens zwei Stationen, vorzugweise zwischen vier und zwölf Stationen, besonders bevorzugt zwischen sechs und zehn Stationen, auf, wobei an jeder der Stationen insbesondere zumindest ein Teil der jeweiligen für die Behandlung der mindestens zwei zu behandelnden Personen erforderlichen Arbeitsschritte durchgeführt werden. Zusätzlich oder alternativ ist das Transportsystem dazu eingerichtet, zumindest einen Teil, vorzugweise jede, der mindestens zwei zu behandelnden Personen mit jedem Takt zur entsprechend nächsten Station zu bewegen.

Weiter zusätzlich oder alternativ dauert ein Takt maximal 30 Minuten, vorzugsweise zwischen 5 und 25 Minuten, besonders bevorzugt zwischen 10 und 20 Minuten. Vorteilhafterweise kann eine derartige Taktung insbesondere im Rahmen standardisierter Eingriffe sowohl die Zeit- als auch die Kosteneffizienz deutlich erhöhen.

Gemäß einer weiteren bevorzugten Ausführungsform des ersten Aspekts der Erfindung umfasst der mindestens eine sterile Bereich mindestens eine Station, vorzugsweise zwischen zwei und sechs Stationen. Zusätzlich oder alternativ umfasst der mindestens eine unsterile Bereich mindestens eine Station, vorzugsweise zwischen zwei und sechs Stationen, besonders bevorzugt zwischen drei und fünf Stationen.

Weiter zusätzlich oder alternativ werden die Stationen gemäß einer definierten Reihenfolge von den mindestens zwei zu behandelnden Personen durchlaufen, wobei, im Falle von mindestens drei Stationen, die entsprechend erste Station und die entsprechend letzte Station gemäß dieser definierten Reihenfolge insbesondere von dem mindestens einen unsterilen Bereich umfasst werden.

Vorteilhafterweise sind die entsprechenden Stationen, die von dem sterilen Bereich umfasst werden, derart angeordnet, dass diese gemäß der Reihenfolge zusammenhängend sind. Hierdurch kann das Transportsystem auf besonders kosteneffiziente Weise realisiert werden. Gemäß einer weiteren bevorzugten Ausführungsform des ersten Aspekts der Erfindung weist das Transportsystem mindestens einen Transportweg, insbesondere mindestens einen Transportweg aufweisend eine Schienen-Anordnung und/oder eine Förderband-Anordnung, auf.

Zusätzlich oder alternativ weist das Transportsystem mindestens zwei Beförderungsmittel, vorzugsweise mindestens zwei Operationstische, besonders bevorzugt mindestens zwei bewegliche Operationstische, zur Beförderung der mindestens zwei zu behandelnden Personen auf.

Weiter zusätzlich oder alternativ ist das Transportsystem dazu eingerichtet, die mindestens zwei Beförderungsmittel über den mindestens einen Transportweg, insbesondere mittels elektrischem Antrieb, zu bewegen. Vorteilhafterweise kann das Transportsystem so auf besonders schnelle und einfache Weise realisiert werden, was zu einer hohen Zeit- und Kostenersparnis führt.

Weiter zusätzlich oder alternativ weist mindestens eines, insbesondere jedes, der mindestens zwei Beförderungsmittel eine Aufbewahrungs- und/oder Halte-Einheit, vorzugweise aufweisend eine Schienen-Anordnung, auf, wobei die Aufbewahrungs- und/oder Halte-Einheit dazu eingerichtet ist, die für die jeweils vollständige oder zumindest einen wesentlichen Teil der Behandlungsdauer der mindestens zwei zu behandelnden Personen erforderlichen Vorrichtungen und/oder Materialien, vorzugsweise mindestens eine Vorrichtung zur Überwachung der jeweiligen Vitalfunktionen der mindestens zwei zu behandelnden Personen, aufzunehmen. Vorteilhafterweise sind derartige Vorrichtungen und/oder Materialien für das medizinische Personal jederzeit verfügbar und erreichbar, wodurch sowohl die Sicherheit des Patienten als auch die Zeit- und die Kosteneffizienz erhöht werden.

Gemäß einem zweiten Aspekt der Erfindung wird ein Transportverfahren geschaffen, wobei das Transportverfahren den folgenden Schritte aufweist: Bewegen von mindestens zwei zu behandelnden Personen zumindest teilweise durch das Innere eines Operationssaals gemäß einem Takt. Vorteilhafterweise kann so die Auslastung im Operationssaal sehr stark erhöht werden, wodurch Operationen bzw. chirurgisch Eingriffe auf besonders zeit- und kosteneffiziente Weise durchgeführt werden können.

Ferner gelten alle bevorzugten Ausführungsformen des ersten Aspekts der Erfindung für den zweiten Aspekt der Erfindung in analoger Weise.

### KURZBESCHREIBUNG DER ZEICHNUNGEN

Nachfolgend wird eine detaillierte beispielhafte Beschreibung einiger Ausführungsformen der Erfindung unter Bezugnahme auf die Figuren der Zeichnung gegeben. Dabei zeigt:
- Fig. 1: eine erste beispielhafte Ausführungsform eines erfindungsgemäßen Transportsystems;
- Fig. 2: eine zweite beispielhafte Ausführungsform eines erfindungsgemäßen Transportsystems; und
- Fig. 3: ein beispielhaftes Flussdiagramm einer Ausführungsform des erfindungsgemäßen Transportverfahrens.

### BESCHREIBUNG BEVORZUGTER AUSFÜHURNGSFORMEN

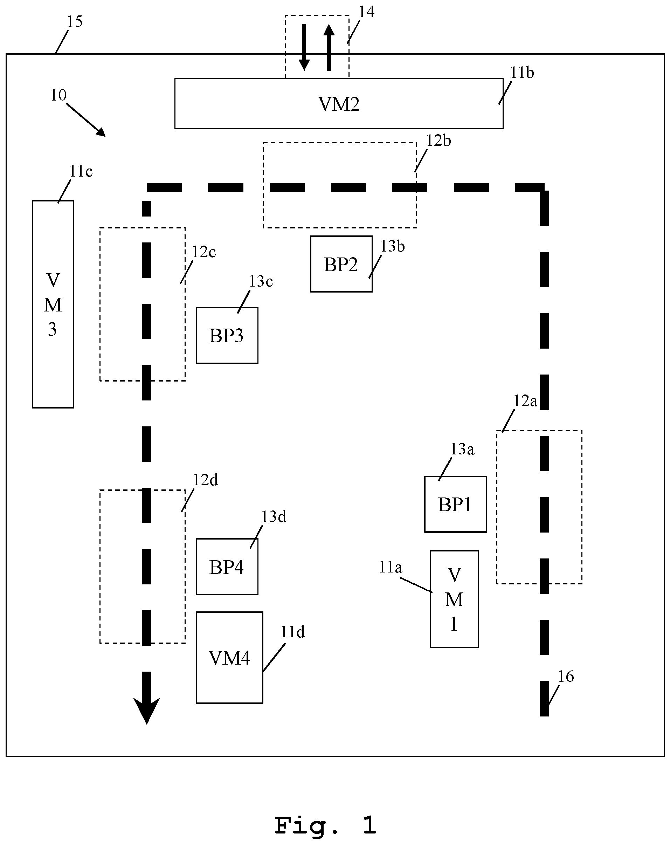

Fig. 1 illustriert eine erste beispielhafte Ausführungsform eines erfindungsgemäßen Transportsystems 10 für einen Operationssaal 15. Hierbei ist das Transportsystem 10 dazu eingerichtet, mindestens zwei, exemplarisch vier, zu behandelnde Personen jeweils zumindest teilweise, exemplarisch vollständig, durch das Innere des Operationssaals 15 gemäß einem Takt zu bewegen.

Ferner weist das Transportsystem 10 gemäß Fig. 1 exemplarisch einen Transportweg 16, insbesondere einen Transportweg aufweisend eine Schienen-Anordnung und/oder eine Förderband-Anordnung, auf. Zudem erkennt man, dass das Transportsystem 10 exemplarisch vier Beförderungsmittel 12a, 12b, 12c, 12d, vorzugsweise vier Operationstische, besonders bevorzugt vier bewegliche Operationstische, zur Beförderung der vier zu behandelnden Personen aufweist.

Außerdem ist das Transportsystem 10 vorzugsweise dazu eingerichtet, die vier Beförderungsmittel 12a, 12b, 12c, 12d über den Transportweg 16, insbesondere mittels elektrischem Antrieb, zu bewegen. Im Falle eines Antriebs, insbesondere des elektrischen Antriebs, kann dieser Antrieb dazu eingerichtet sein, den Transportweg 16 bzw. beispielsweise eine Förderband-Anordnung des Transportwegs 16 entsprechend anzutreiben. Ferner kann dieser Antrieb dazu eingerichtet sein, das entsprechende Beförderungsmittel der im Beispiel vier Beförderungsmittel 12a, 12b, 12c, 12d anzutreiben. Es sei darauf hingewiesen, dass auch eine Kombination dieser beiden Möglichkeiten verwendet werden kann.

Dementsprechend mag der mindestens eine, exemplarisch der Transportweg 16, und/oder mindestens eines der mindestens zwei, exemplarisch vier, Beförderungsmittel 12a, 12b, 12c, 12d einen Antrieb, insbesondere einen elektrischen Antrieb, aufweisen.

Es mag besonders vorteilhaft sein, wenn mindestens eines, insbesondere jedes, der vier Beförderungsmittel 12a, 12b, 12c, 12d eine Aufbewahrungs- und/oder Halte-Einheit, vorzugweise aufweisend eine Schienen-Anordnung, aufweist. Dabei ist die Aufbewahrungs- und/oder Halte-Einheit dazu eingerichtet, die für die jeweils vollständige oder zumindest einen wesentlichen Teil der Behandlungsdauer der entsprechenden der im Beispiel vier zu behandelnden Personen erforderlichen Vorrichtungen und/oder Materialien, vorzugsweise mindestens eine Vorrichtung zur Überwachung der jeweiligen Vitalfunktionen der entsprechenden der vier zu behandelnden Personen, aufzunehmen.

Zusätzlich oder alternativ ist es vorteilhaft, wenn das Transportsystem 10 mindestens einen zusätzlichen Transportweg, insbesondere mindestens einen zusätzlichen Transportweg aufweisend eine Schienen-Anordnung und/oder eine Förderband-Anordnung, aufweist. Dieser mindestens eine zusätzliche Transportweg kann insbesondere dazu eingerichtet sein, die für die jeweils vollständige oder zumindest einen wesentlichen Teil der Behandlungsdauer der entsprechenden der vier zu behandelnden Personen erforderlichen Vorrichtungen und/oder Materialien, vorzugsweise mindestens eine Vorrichtung zur Überwachung der jeweiligen Vitalfunktionen der entsprechenden der vier zu behandelnden Personen, zu transportieren. Hierfür kann der mindestens eine zusätzliche Transportweg einen Antrieb, insbesondere einen elektrischen Antrieb, aufweisen.

Es mag besonders vorteilhaft sein, wenn das Transportsystem 10 dazu eingerichtet ist, die im Beispiel vier zu behandelnden Personen derart zu bewegen, dass jeweils zumindest temporär eine Relativbewegung zwischen zumindest einem Teil der im Beispiel vier zu behandelnden Personen und zumindest einem Teil zur entsprechenden Behandlung erforderlicher Vorrichtungen und/oder Materialien 11a, 11b, 11c, 11d entsteht.

Hierbei kann zumindest ein Teil der zur entsprechenden Behandlung erforderlichen Vorrichtungen und/oder Materialien 11a, 11b, 11c, 11d zumindest teilweise im Inneren des Operationssaals 15 statisch oder nahezu statisch lokalisiert sein. Dementsprechend kann das Transportsystem 10 zusätzlich oder alternativ dazu eingerichtet sein, zumindest einen Teil der zur entsprechenden Behandlung erforderlichen Vorrichtungen und/oder Materialien 11a, 11b, 11c, 11d zumindest teilweise im Inneren des Operationssaals 15 zu bewegen.

Hierzu kann beispielsweise der oben genannte mindestens eine zusätzliche Transportweg verwendet werden. Ferner kann mindestens ein weiterer zusätzlicher Transportweg geschaffen werden. Dieser mindestens eine weitere zusätzliche Transportweg mag hinsichtlich seiner Eigenschaften denen des mindestens einen zusätzlichen Transportwegs in analoger Weise zumindest teilweise entsprechen.

Ferner ist es besonders vorteilhaft, wenn das Transportsystem 10 dazu eingerichtet ist, die mindestens zwei zu behandelnden Personen derart zu bewegen, dass zwischen zumindest einem Teil der mindestens zwei zu behandelnden Personen und mindestens einer behandelnden Person (der exemplarischen vier behandelnden Personen 13a, 13b, 13c, 13d) keine oder eine vernachlässigbare Relativbewegung entsteht.

Es sei angemerkt, dass beispielsweise zumindest ein Teil der behandelnden Personen 13a, 13b, 13c, 13d der entsprechenden zu behandelnden Person zumindest teilweise, insbesondere zumindest teilweise während der entsprechenden Bewegung über den Transportweg 16, folgen mag.

Zusätzlich oder alternativ kann das Transportsystem 10 dazu eingerichtet sein, die mindestens zwei zu behandelnden Personen derart zu bewegen, dass jeweils zumindest temporär eine Relativbewegung zwischen mindestens einer behandelnden Person (der beispielhaften vier behandelnden Personen 13a, 13b, 13c, 13d) und zumindest einem Teil der zur entsprechenden Behandlung erforderlichen Vorrichtungen und/oder Materialien 11a, 11b, 11c, 11d entsteht. Die vorgenannte Relativbewegung mag insbesondere der oben genannten Relativbewegung zwischen zumindest einem Teil der mindestens zwei zu behandelnden Personen und zumindest einem Teil zur entsprechenden Behandlung erforderlicher Vorrichtungen und/oder Materialien 11a, 11b, 11c, 11d zumindest annähernd entsprechen.

Zudem sei angemerkt, dass mindestens eine behandelnde Person (der beispielhaften vier behandelnden Personen 13a, 13b, 13c, 13d) im Inneren des Operationssaals 15 statisch oder nahezu statisch lokalisiert sein kann.

Ferner mag das Transportsystem 10 dazu eingerichtet sein, zumindest einen Teil zur entsprechenden Behandlung erforderlicher Vorrichtungen und/oder Materialien 11a, 11b, 11c, 11d derart zu bewegen, dass jeweils zumindest temporär eine Relativbewegung zwischen zumindest einem Teil der mindestens zwei zu behandelnden Personen und zumindest einem Teil zur entsprechenden Behandlung erforderlicher Vorrichtungen und/oder Materialien 11a, 11b, 11c, 11d entsteht. Hierfür kann beispielsweise der oben genannte mindestens eine zusätzliche Transportweg bzw. der mindestens eine weitere zusätzliche Transportweg verwendet werden.

Zusätzlich oder alternativ kann das Transportsystem 10 dazu eingerichtet sein, zumindest einen Teil zur entsprechenden Behandlung erforderlicher Vorrichtungen und/oder Materialien 11a, 11b, 11c, 11d derart zu bewegen, dass jeweils zumindest temporär eine Relativbewegung zwischen mindestens einer behandelnden Person (der exemplarischen vier behandelnden Personen 13a, 13b, 13c, 13d) und zumindest einem Teil der zur entsprechenden Behandlung erforderlichen Vorrichtungen und/oder Materialien 11a, 11b, 11c, 11d entsteht. Auch hierfür kann beispielsweise der oben genannte mindestens eine zusätzliche Transportweg bzw. der mindestens eine weitere zusätzliche Transportweg verwendet werden.

In diesem Kontext mag es besonders vorteilhaft sein, wenn die entsprechende Bewegungsrichtung des zumindest einen Teils der zur entsprechenden Behandlung erforderlichen Vorrichtungen und/oder Materialien 11a, 11b, 11c, 11d senkrecht oder nahezu senkrecht zu der entsprechenden Bewegungsrichtung mindestens eines Teils der mindestens zwei, exemplarisch vier, zu behandelnden Personen ist.

Insbesondere die vorgenannte Orthogonalität wird durch den Block 14 in der Fig. 1 illustriert. Es sei angemerkt, dass die Zuführung der zur entsprechenden Behandlung erforderlichen Vorrichtungen und/oder Materialien, exemplarisch 11b, vorteilhafterweise an einer Seite des entsprechenden Beförderungsmittels, exemplarisch 12b, erfolgen mag, an der sich die entsprechend behandelnde Person, exemplarisch 13b, während der entsprechenden Behandlung großenteils aufhält. Besonders vorteilhaft ist es, wenn die vorgenannte Zuführung an der Seite des entsprechenden Beförderungsmittels erfolgt, welche der Seite, an der sich die entsprechend behandelnde Person während der entsprechenden Behandlung großenteils aufhält, gegenüberliegt, wie in Fig. 1 dargestellt.

Zusätzlich oder alternativ kann diese Zuführung der zur entsprechenden Behandlung erforderlichen Vorrichtungen und/oder Materialien durch eine Öffnung oder eine Schleuse im Operationssaal 15 erfolgen. Ferner kann für diese Zuführung beispielsweise der oben genannte mindestens eine zusätzliche Transportweg bzw. der mindestens eine weitere zusätzliche Transportweg verwendet werden. Zusätzlich oder alternativ können weitere Transportwege geschaffen werden.

Es sei darauf hingewiesen, dass die bei der entsprechenden Behandlung benutzten Vorrichtungen und/oder Materialien in analoger Weise abgeführt werden können. Hierfür kann beispielsweise jeweils ein entsprechend paralleler oder annähernd paralleler Transportweg geschaffen werden. Ein besonderer Vorteil kann sich ergeben, wenn die Abführungen der benutzten Vorrichtungen und/oder Materialien auf einer anderen Ebene, insbesondere auf einer anderen Ebene bezüglich der Horizontalen, erfolgt. Beispielweise kann die Zuführung ungefähr auf Höhe des entsprechenden Beförderungsmittels bzw. Operationstisches erfolgen, während die Abführung unterhalb dieser Höhe erfolgt.

Zusätzlich oder alternativ mag die entsprechende Bewegungsrichtung des zumindest einen Teils der zur entsprechenden Behandlung erforderlichen Vorrichtungen und/oder Materialien 11a, 11b, 11c, 11d senkrecht oder nahezu senkrecht zu der entsprechenden Bewegungsrichtung mindestens eines Teils der mindestens einen, exemplarisch vier, behandelnden Personen 13a, 13b, 13c, 13d sein.

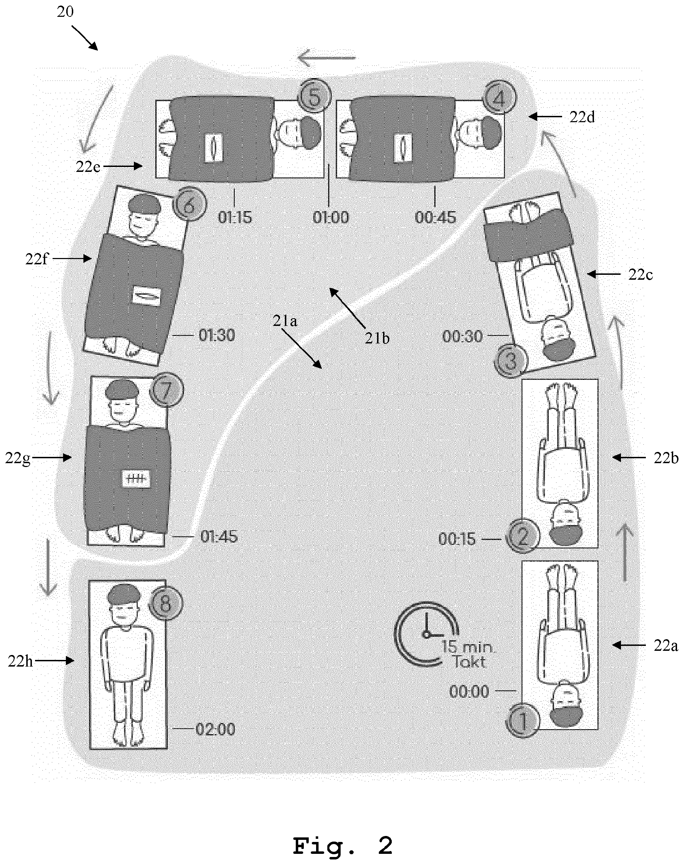

Gemäß Fig. 1 weist das Transportsystem 10 ferner einen sterilen Bereich im Inneren des Operationssaals 15 auf. Zusätzlich oder alternativ kann das Transportsystem mindestens einen unsterilen Bereich, vorzugsweise mindestens einen unsterilen Bereich außerhalb des Operationssaals 15, aufweisen, was Fig. 2 noch verdeutlichen wird.

Außerdem lässt Fig. 1 erkennen, dass das Transportsystem 10 vier Stationen (eine Station umfasst beispielhaft die entsprechenden zur Behandlung erforderlichen Vorrichtungen und/oder Materialien 11a, 11b, 11c, 11d) aufweist, wobei an jeder der Stationen insbesondere zumindest ein Teil der jeweiligen für die Behandlung der mindestens zwei zu behandelnden Personen erforderlichen Arbeitsschritte durchgeführt werden.

Ferner ist das Transportsystem 10 dazu eingerichtet, zumindest einen Teil, vorzugweise jede, der vier zu behandelnden Personen mit jedem Takt zur entsprechend nächsten Station zu bewegen. In diesem Kontext dauert ein Takt maximal 30 Minuten, vorzugsweise zwischen 5 und 25 Minuten, besonders bevorzugt zwischen 10 und 20 Minuten, beispielsweise zwischen 14 und 16 Minuten.

Weiterhin sei darauf hingewiesen, dass die Pfeilrichtung des Transportwegs 16 anzeigt, dass die Stationen gemäß einer definierten Reihenfolge von den vier zu behandelnden Personen durchlaufen werden.

Wie bereits oben angedeutet, zeigt Fig. 2 eine zweite beispielhafte Ausführungsform des erfindungsgemäßen Transportsystems 20, die einen unsterilen Bereich 21a sowie einen sterilen Bereich 21b aufweist.

Ferner weist diese zweite Ausführungsform 20 insgesamt acht Stationen 22a, 22b, 22c, 22d, 22e, 22f, 22g, 22h auf, die gemäß der Reihenfolge 1-8 von den zu behandelnden Personen durchlaufen werden. Beispielhaft dauert ein Takt 15 Minuten. Dementsprechend dauert die Behandlung einer zu behandelnden Person bzw. eines Patienten in diesem Beispiel zwei Stunden.

Weiterhin weist der unsterile Bereich 21a vier Stationen, insbesondere die ersten drei Stationen 22a, 22b, 22c sowie die letzte Station 22h gemäß der Reihenfolge, auf. Der sterile Bereich 21b weist ebenfalls vier Stationen, insbesondere die zusammenhängenden Stationen 22d, 22e, 22f, 22g gemäß der Reihenfolge, auf.

Im Folgenden sei angenommen, dass es sich bei der im Rahmen der Ausführungsform gemäß Fig. 2 durchgeführten Operation um eine Operation der menschlichen Hüfte handelt. In diesem Kontext werden die Stationen bzw. die entsprechenden Arbeitsschritte, die an dieser durchgeführt werden, sowie die etwaige benötigte Vorrichtungen und/oder Materialien beispielhaft beschrieben.

An der ersten Station 22a bzw. im ersten Arbeitstakt wird die entsprechende Anästhesie der zu behandelnden Person bzw. des Patienten vorbereitet. Die erste Station mag zumindest einen Teil der folgenden Vorrichtungen bzw. medizinischen Geräte aufweisen: Anästhesiesystem, patientennahe Atemgasmessung, Pulsoximeter, EKG-Monitor (Elektrokardiogramm-Monitor), Blutdruck-Messung, Körpertemperatur-Messung, Defibrillator, Relaxometer, Blutzucker-Messgerät, Anästhesie-Beatmungsgerät, oszillometrische Blutdruck-Messung. Das vorgenannte Anästhesiesystem mag beispielsweise zumindest einen Teil der folgenden Komponenten aufweisen: Druckbegrenzung, Kapnometrie, Sauerstoff-Überwachungsgerät, Überwachung des Exspirationsvolumens, Diskonnektions-Alarm, Apnoe-Alarm, Anästhesiegasfortleitungssystem, Anäthesiemittel-Überwachungsgerät.

Es sei darauf hingewiesen, dass zumindest ein Teil der vorgenannten Vorrichtungen über mehrere Stationen hinweg mitgenommen werden kann. An der folgenden Station 22b bzw. in einem zweiten Arbeitstakt wird die Anästhesie des Patienten entsprechend eingeleitet.

An der dritten Stationen 22c bzw. im dritten Arbeitstakt wird der Patient entsprechend gelagert und weiter für die Operation vorbereitet. Hierbei kommen beispielsweise an die körperlichen Ausmaße des Patienten angepasste Polster zum Einsatz. Ferner werden beispielhaft die entsprechend zu setzenden Schnitte markiert. Es mag besonders vorteilhaft sein, wenn mindestens eine behandelnde Person den entsprechenden Patienten von der ersten Station 22a bis zur dritten Station 22c begleitet.

An der vierten Station 22d bzw. im vierten Arbeitstakt erfolgt beispielhaft der chirurgische Schnitt bzw. das Freilegen des Operationsfeldes. Hierbei werden beispielweise die entsprechenden Schnitte in Haut und Muskulatur gesetzt. Für Knochenschnitte mag außerdem eine Schnittleere befestigt werden. Die Knochenschnitte mögen zudem entsprechend durchgeführt werden.

An der fünften Station 22e bzw. im fünften Arbeitstakt wird beispielhaft die defekte Hüfte entfernt. Anschließend wird im sechsten Arbeitstakt bzw. an der sechsten Station 22f das Implantat bzw. die Implantate, exemplarisch Hüft-Implantat bzw. Femurschaft für Hüfte, entsprechend eingesetzt. Ferner werden entsprechende Gegenstücke des Implantats bzw. der Implantate, beispielsweise Pfanne für Hüfte, eingesetzt.

Es sei darauf hingewiesen, dass mindestens eine der Stationen 22a, 22b, 22c, 22d, 22e, 22f, 22g, 22h, vorzugweise die sechste Station 22f, eine RöntgenEinrichtung, insbesondere einen C-Bogen zum Röntgen, und/oder einen Magnetresonanztomographen aufweisen mag. An der siebten Station 22f bzw. im siebten Arbeitstakt wird das Operationsfeld beispielsweise mittels Nähen verschlossen.

Es sei angemerkt, dass es besonders vorteilhaft sein mag, wenn mindestens eine behandelnde Person den entsprechenden Patienten von der vierten Station 22d bis zur siebten Station 22g begleitet. Diese mindestens eine behandelnde Person mag vorzugsweise nicht der mindestens einen behandelnden Person entsprechen, die den entsprechenden Patienten bei den Stationen 22a bis 22c begleitet.

Weiterhin erfolgt an der achten Station 22h bzw. im achten Arbeitstakt die entsprechende Anästhesie-Ausleitung. Ferner kann beispielsweise die entsprechende Umlagerung des Patienten erfolgen. Es sei darauf hingewiesen, dass es besonders vorteilhaft sein mag, wenn mindestens eine behandelnde Person den entsprechenden Patienten an dieser Station 22h versorgt. Diese mindestens eine behandelnde Person mag vorzugsweise nicht der mindestens einen behandelnden Person entsprechen, die den entsprechenden Patienten bei den Stationen 22d bis 22g begleitet.

Zusätzlich oder alternativ sind die beiden folgenden Alternativen auf ihre Art jeweils vorteilhaft: Diese mindestens eine behandelnde Person (an der achten Station 22h) mag nicht der mindestens einen behandelnden Person entsprechen, die den entsprechenden Patienten bei den Stationen 22a bis 22c begleitet, oder mag der mindestens einen behandelnden Person entsprechen, die den entsprechenden Patienten bei den Stationen 22a bis 22c begleitet.

Ferner sei angemerkt, dass die Reihenfolge des Durchlaufens der Stationen 22a, 22b, 22c, 22d, 22e, 22f, 22g, 22h nicht immer zwingend eingehalten werden muss. Beispielsweise im Falle etwaiger Komplikationen ist es nicht nur möglich, sondern wahrscheinlich auch erforderlich, von der vorgegebenen Reihenfolge abzuweichen. Zudem kann auch ein iteratives Vorgehen bei der Operation, beispielsweise Kontrollieren der Lage des Implantats mittels bildgebendem Verfahren und eventuelles Nachjustieren des Implantats, ein Abweichen von der vordefinierten Reihenfolge der Stationen erfordern.

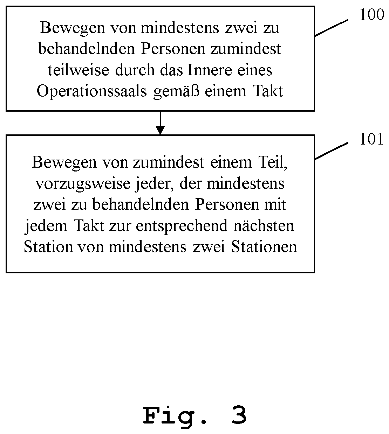

Abschließend zeigt Fig. 3 ein beispielhaftes Flussdiagramm des erfindungsgemäßen Transportverfahrens. In einem ersten Schritt 100 werden mindestens zwei zu behandelnden Personen zumindest teilweise durch das Innere eines Operationssaals gemäß einem Takt bewegt. Gemäß einem zweiten Schritt 101 wird dann zumindest ein Teil, vorzugsweise jede, der mindestens zwei zu behandelnden Personen mit jedem Takt zur entsprechend nächsten Station von mindestens zwei Stationen bewegt.

Es mag besonders vorteilhaft sein, wenn die mindestens zwei zu behandelnden Personen derart bewegt werden, dass jeweils zumindest temporär eine Relativbewegung zwischen zumindest einem Teil der mindestens zwei zu behandelnden Personen und zumindest einem Teil zur entsprechenden Behandlung erforderlicher Vorrichtungen und/oder Materialien entsteht.

Zusätzlich oder alternativ mag zumindest ein Teil der zur entsprechenden Behandlung erforderlichen Vorrichtungen und/oder Materialien zumindest teilweise im Inneren des Operationssaals statisch oder nahezu statisch lokalisiert sein.

Ferner mag es vorteilhaft sein, wenn die mindestens zwei zu behandelnden Personen derart bewegt werden, dass zwischen zumindest einem Teil der mindestens zwei zu behandelnden Personen und mindestens einer behandelnden Person keine oder eine vernachlässigbare Relativbewegung entsteht.

Zusätzlich oder alternativ können die mindestens zwei zu behandelnden Personen derart bewegt werden, dass jeweils zumindest temporär eine Relativbewegung zwischen mindestens einer behandelnden Person und zumindest einem Teil der zur entsprechenden Behandlung erforderlichen Vorrichtungen und/oder Materialien entsteht, wobei im Falle der oben genannten Relativbewegung zwischen zumindest einem Teil der mindestens zwei zu behandelnden Personen und zumindest einem Teil zur entsprechenden Behandlung erforderlicher Vorrichtungen und/oder Materialien, die vorgenannte Relativbewegung insbesondere dieser oben genannten Relativbewegung zumindest annähernd entspricht.

Ferner sei darauf hingewiesen, dass zusätzlich oder alternativ mindestens eine behandelnde Person im Inneren des Operationssaals statisch oder nahezu statisch lokalisiert sein kann. Weiterhin mag es besonders vorteilhaft sein, wenn zumindest ein Teil zur entsprechenden Behandlung erforderlicher Vorrichtungen und/oder Materialien derart bewegt wird, dass jeweils zumindest temporär eine Relativbewegung zwischen zumindest einem Teil der mindestens zwei zu behandelnden Personen und zumindest einem Teil zur entsprechenden Behandlung erforderlicher Vorrichtungen und/oder Materialien entsteht.

Zusätzlich oder alternativ kann zumindest ein Teil zur entsprechenden Behandlung erforderlicher Vorrichtungen und/oder Materialien derart bewegt werden, dass jeweils zumindest temporär eine Relativbewegung zwischen mindestens einer behandelnden Person und zumindest einem Teil der zur entsprechenden Behandlung erforderlichen Vorrichtungen und/oder Materialien entsteht.

In diesem Zusammenhang kann es besonders vorteilhaft sein, wenn die entsprechende Bewegungsrichtung des zumindest einen Teils der zur entsprechenden Behandlung erforderlichen Vorrichtungen und/oder Materialien senkrecht oder nahezu senkrecht zu der entsprechenden Bewegungsrichtung mindestens eines Teils der mindestens zwei zu behandelnden Personen ist.

Ferner sei darauf hingewiesen, dass die Bewegung, insbesondere die Bewegung der mindestens zwei zu behandelnden Personen, durch mindestens einen sterilen Bereich, vorzugsweise mindestens einen sterilen Bereich im Inneren des Operationssaals, erfolgt. Zusätzlich oder alternativ mag die Bewegung, insbesondere die Bewegung der mindestens zwei zu behandelnden Personen, durch mindestens einen unsterilen Bereich, vorzugsweise mindestens einen unsterilen Bereich außerhalb des Operationssaals, erfolgen.

Insbesondere bezüglich des oben genannten Schritts 101 sei angemerkt, dass die Bewegung, insbesondere die Bewegung der mindestens zwei zu behandelnden Personen, durch mindestens zwei Stationen, vorzugweise zwischen vier und zwölf Stationen, besonders bevorzugt zwischen sechs und zehn Stationen, z.B. acht Stationen, erfolgen kann, wobei an jeder der Stationen insbesondere zumindest ein Teil der jeweiligen für die Behandlung der mindestens zwei zu behandelnden Personen erforderlichen Arbeitsschritte durchgeführt werden.

Zusätzlich oder alternativ sei hinsichtlich des Takts darauf hingewiesen, dass ein Takt maximal 30 Minuten, vorzugsweise zwischen 5 und 25 Minuten, besonders bevorzugt zwischen 10 und 20 Minuten, beispielsweise zwischen 14 und 16 Minuten, dauert.

Weiterhin mag es besonders vorteilhaft sein, wenn der mindestens eine sterile Bereich mindestens eine Station, vorzugsweise zwischen zwei und sechs Stationen, besonders bevorzugt zwischen drei und fünf Stationen, beispielsweise vier Stationen, umfasst.

Zusätzlich oder alternativ kann der mindestens eine unsterile Bereich mindestens eine Station, vorzugsweise zwischen zwei und sechs Stationen, besonders bevorzugt zwischen drei und fünf Stationen, beispielsweise vier Stationen, umfassen.

Weiter zusätzlich oder alternativ können die Stationen gemäß einer definierten Reihenfolge von den mindestens zwei zu behandelnden Personen durchlaufen werden, wobei, im Falle von mindestens drei Stationen, die entsprechend erste Station und die entsprechend letzte Station gemäß dieser definierten Reihenfolge insbesondere von dem mindestens einen unsterilen Bereich umfasst werden.

Es sei außerdem darauf hingewiesen, dass die Bewegung, insbesondere die Bewegung der mindestens zwei zu behandelnden Personen, über mindestens einen Transportweg, vorzugsweise mindestens einen Transportweg aufweisend eine Schienen-Anordnung und/oder eine Förderband-Anordnung, erfolgen mag. Zusätzlich oder alternativ mögen zur Bewegung, insbesondere zur Bewegung der mindestens zwei zu behandelnden Personen, mindestens zwei Beförderungsmittel, vorzugsweise mindestens zwei Operationstische, besonders bevorzugt mindestens zwei bewegliche Operationstische, verwendet werden.

Weiter zusätzlich oder alternativ mögen die mindestens zwei Beförderungsmittel über den mindestens einen Transportweg, insbesondere mittels elektrischem Antrieb, bewegt werden.

Weiterhin - zusätzlich oder alternativ - kann mindestens eines, insbesondere jedes, der mindestens zwei Beförderungsmittel eine Aufbewahrungs- und/oder Halte-Einheit, vorzugweise aufweisend eine Schienen-Anordnung, aufweisen, wobei die Aufbewahrungs- und/oder Halte-Einheit dazu eingerichtet ist, die für die jeweils vollständige oder zumindest einen wesentlichen Teil der Behandlungsdauer der mindestens zwei zu behandelnden Personen erforderlichen Vorrichtungen und/oder Materialien, vorzugsweise mindestens eine Vorrichtung zur Überwachung der jeweiligen Vitalfunktionen der mindestens zwei zu behandelnden Personen, aufzunehmen.

Die Erfindung ist nicht auf die vorstehend diskutierten Ausführungsbeispiele beschränkt. Alle in der Beschreibung beschriebenen Merkmale oder in den Patentansprüchen beanspruchten Merkmale oder in der Zeichnung gezeichneten Merkmale sind im Rahmen dieser Erfindung beliebig miteinander kombinierbar.

## Patentansprüche

1. Ein Transportsystem (10, 20) für einen Operationssaal (15),
wobei das Transportsystem (10, 20) dazu eingerichtet ist, mindestens zwei zu behandelnde Personen jeweils zumindest teilweise durch das Innere des Operationssaals (15) gemäß einem Takt zu bewegen.

2. Das Transportsystem (10, 20) gemäß Anspruch 1,
wobei das Transportsystem (10, 20) dazu eingerichtet ist, die mindestens zwei zu behandelnden Personen derart zu bewegen, dass jeweils zumindest temporär eine Relativbewegung zwischen zumindest einem Teil der mindestens zwei zu behandelnden Personen und zumindest einem Teil zur entsprechenden Behandlung erforderlicher Vorrichtungen und/oder Materialien (11a, 11b, 11c, 11d) entsteht, und/oder
wobei zumindest ein Teil der zur entsprechenden Behandlung erforderlichen Vorrichtungen und/oder Materialien (11a, 11b, 11c, 11d) zumindest teilweise im Inneren des Operationssaals (15) statisch oder nahezu statisch lokalisiert ist.

3. Das Transportsystem (10, 20) gemäß Anspruch 1 oder 2,
wobei das Transportsystem (10, 20) dazu eingerichtet ist, die mindestens zwei zu behandelnden Personen derart zu bewegen, dass zwischen zumindest einem Teil der mindestens zwei zu behandelnden Personen und mindestens einer behandelnden Person (13a, 13b, 13c, 13d) keine oder eine vernachlässigbare Relativbewegung entsteht, und/oder wobei das Transportsystem (10, 20) dazu eingerichtet ist, die mindestens zwei zu behandelnden Personen derart zu bewegen, dass jeweils zumindest temporär eine Relativbewegung zwischen mindestens einer behandelnden Person (13a, 13b, 13c, 13d) und zumindest einem Teil der zur entsprechenden Behandlung erforderlichen Vorrichtungen und/oder Materialien (11a, 11b, 11c, 11d) entsteht, wobei im Falle des Anspruchs 2, die vorgenannte Relativbewegung insbesondere der Relativbewegung gemäß Anspruch 2 zumindest annähernd entspricht, und/oder
wobei mindestens eine behandelnde Person (13a, 13b, 13c, 13d) im Inneren des Operationssaals (15) statisch oder nahezu statisch lokalisiert ist.

4. Das Transportsystem (10, 20) gemäß einem der Ansprüche 1 bis 3,
wobei das Transportsystem (10, 20) dazu eingerichtet ist, zumindest einen Teil zur entsprechenden Behandlung erforderlicher Vorrichtungen und/oder Materialien (11a, 11b, 11c, 11d) derart zu bewegen, dass jeweils zumindest temporär eine Relativbewegung zwischen zumindest einem Teil der mindestens zwei zu behandelnden Personen und zumindest einem Teil zur entsprechenden Behandlung erforderlicher Vorrichtungen und/oder Materialien (11a, 11b, 11c, 11d) entsteht, und/oder
wobei das Transportsystem (10, 20) dazu eingerichtet ist, zumindest einen Teil zur entsprechenden Behandlung erforderlicher Vorrichtungen und/oder Materialien (11a, 11b, 11c, 11d) derart zu bewegen, dass jeweils zumindest temporär eine Relativbewegung zwischen mindestens einer behandelnden Person (13a, 13b, 13c, 13d) und zumindest einem Teil der zur entsprechenden Behandlung erforderlichen Vorrichtungen und/oder Materialien (11a, 11b, 11c, 11d) entsteht.

5. Das Transportsystem (10, 20) gemäß Anspruch 4,
wobei die entsprechende Bewegungsrichtung des zumindest einen Teils der zur entsprechenden Behandlung erforderlichen Vorrichtungen und/oder Materialien (11a, 11b, 11c, 11d) senkrecht (14) oder nahezu senkrecht zu der entsprechenden Bewegungsrichtung mindestens eines Teils der mindestens zwei zu behandelnden Personen ist.

6. Das Transportsystem (10, 20) gemäß einem der Ansprüche 1 bis 5,
wobei das Transportsystem (10, 20) mindestens einen sterilen Bereich (21b), vorzugsweise mindestens einen sterilen Bereich im Inneren des Operationssaals (15), aufweist, und/oder
wobei das Transportsystem (10, 20) mindestens einen unsterilen Bereich (21a), vorzugsweise mindestens einen unsterilen Bereich außerhalb des Operationssaals (15), aufweist.

7. Das Transportsystem (10, 20) gemäß einem der Ansprüche 1 bis 6,
wobei das Transportsystem (10, 20) mindestens zwei Stationen (22a, 22b, 22c, 22d, 22e, 22f, 22g, 22h), vorzugweise zwischen vier und zwölf Stationen, besonders bevorzugt zwischen sechs und zehn Stationen, aufweist, wobei an jeder der Stationen insbesondere zumindest ein Teil der jeweiligen für die Behandlung der mindestens zwei zu behandelnden Personen erforderlichen Arbeitsschritte durchgeführt werden, und/oder
wobei das Transportsystem (10, 20) dazu eingerichtet ist, zumindest einen Teil, vorzugweise jede, der mindestens zwei zu behandelnden Personen mit jedem Takt zur entsprechend nächsten Station zu bewegen, und/oder wobei ein Takt maximal 30 Minuten, vorzugsweise zwischen 5 und 25 Minuten, besonders bevorzugt zwischen 10 und 20 Minuten, dauert.

8. Das Transportsystem (10, 20) gemäß der Ansprüche 6 und 7,
wobei der mindestens eine sterile Bereich (21b) mindestens eine Station (22d, 22e, 22f, 22g), vorzugsweise zwischen zwei und sechs Stationen, besonders bevorzugt zwischen drei und fünf Stationen, umfasst, und/oder wobei der mindestens eine unsterile Bereich (21a) mindestens eine Station (22a, 22b, 22c, 22h), vorzugsweise zwischen zwei und sechs Stationen, besonders bevorzugt zwischen drei und fünf Stationen, umfasst, und/oder wobei die Stationen gemäß einer definierten Reihenfolge (1, 2, 3, 4, 5, 6, 7, 8) von den mindestens zwei zu behandelnden Personen durchlaufen werden, wobei, im Falle von mindestens drei Stationen, die entsprechend erste Station (22a) und die entsprechend letzte Station (22h) gemäß dieser definierten Reihenfolge (1, 2, 3, 4, 5, 6, 7, 8) insbesondere von dem mindestens einen unsterilen Bereich (21a) umfasst werden.

9. Das Transportsystem (10, 20) nach einem der Ansprüche 1 bis 8,
wobei das Transportsystem (10, 20) mindestens einen Transportweg (16), insbesondere mindestens einen Transportweg aufweisend eine Schienen-Anordnung und/oder eine Förderband-Anordnung, aufweist, und/oder wobei das Transportsystem (10, 20) mindestens zwei Beförderungsmittel (12a, 12b, 12c, 12d), vorzugsweise mindestens zwei Operationstische, besonders bevorzugt mindestens zwei bewegliche Operationstische, zur Beförderung der mindestens zwei zu behandelnden Personen aufweist, und/oder
wobei das Transportsystem (10, 20) dazu eingerichtet ist, die mindestens zwei Beförderungsmittel (12a, 12b, 12c, 12d) über den mindestens einen Transportweg (16), insbesondere mittels elektrischem Antrieb, zu bewegen, und/oder
wobei mindestens eines, insbesondere jedes, der mindestens zwei Beförderungsmittel (12a, 12b, 12c, 12d) eine Aufbewahrungs- und/oder Halte-Einheit, vorzugweise aufweisend eine Schienen-Anordnung, aufweist, wobei die Aufbewahrungs- und/oder Halte-Einheit dazu eingerichtet ist, die für die jeweils vollständige oder zumindest einen wesentlichen Teil der Behandlungsdauer der mindestens zwei zu behandelnden Personen erforderlichen Vorrichtungen und/oder Materialien, vorzugsweise mindestens eine Vorrichtung zur Überwachung der jeweiligen Vitalfunktionen der mindestens zwei zu behandelnden Personen, aufzunehmen.

10. Ein Transportverfahren,
wobei das Transportverfahren den folgenden Schritt (100) aufweist:
Bewegen von mindestens zwei zu behandelnden Personen zumindest teilweise durch das Innere eines Operationssaals (15) gemäß einem Takt, insbesondere mittels eines Transportsystems (10, 20) nach einem der Ansprüche 1 bis 9.
